# EUROPEAN PATENT SPECIFICATION

(11) **EP 2 832 344 B1**
(45) Date of publication and mention of the grant of the patent: **01.08.2018**
(21) Application number: 13768078.1
(22) Date of filing: 26.02.2013
(51) Int. Cl.: A61K 8/34, A61K 8/35, A61K 31/01, A61K 31/015, A61K 31/05, A61K 31/122, A61P 17/00, A61Q 19/00, A61Q 19/08, A61K 8/31, A61Q 19/02, A61K 9/00, A23L 33/10, A23L 33/105, A23L 33/155, A23L 5/44

(54) **COMPOSITION, AND EXTERNAL PREPARATION FOR THE SKIN OR FUNCTIONAL FOOD EACH CONTAINING SAID COMPOSITION**
ZUSAMMENSETZUNG UND EXTERNES PRÄPARAT FÜR DIE HAUT ODER FUNKTIONSNAHRUNG MIT DIESER ZUSAMMENSETZUNG
COMPOSITION, ET PRÉPARATION EXTERNE POUR LA PEAU OU ALIMENT FONCTIONNEL CONTENANT CHACUN LADITE COMPOSITION

(30) Priority: 28.03.2012 JP 2012074015; 08.01.2013 JP 2013001065
(43) Date of publication of application: 04.02.2015
(73) Proprietor: FUJIFILM Corporation, Tokyo 106-8620 (JP)
(72) Inventor: MURAGUCHI Taichi, Ashigarakami-gun Kanagawa 258-8577 (JP); THIELE Tomoko, Ashigarakami-gun Kanagawa 258-8577 (JP)
(74) Representative: Hoffmann Eitle
(86) International application number: PCT/JP2013/054979
(87) International publication number: WO 2013/146018

(56) References cited:
- WO-A1-02/13835
- WO-A1-02/098384
- WO-A1-2010/112071
- WO-A1-2011/051483
- WO-A2-01/43705
- TW-A- 201 127 417
- US-A1- 2009 263 367
- US-A1- 2011 274 680
- DATABASE GNPD [Online] MINTEL; September 2012 (2012-09), Anonymus: "Essence Destiny", XP002742844, retrieved from www.gnpd.com Database accession no. 2008904
- NOEVIR: 'BioEssentials Supplement' December 2011, XP055159626 Retrieved from the Internet: <URL:http://www.gnpd.com/sinatra/ home.html> ID:1698967> [retrieved on 2013-04-26]
- YAMASHITA. E. FRAGRANCE JOURNAL vol. 34, no. 3, March 2006, pages 21 - 27, XP008174298
- SOMEYA, K. FRAGRANCE JOURNAL vol. 22, no. 10, October 1994, pages 93 - 101, XP008174299

## Description

### BACKGROUND OF THE INVENTION

### 1. Field of the Invention

The present invention relates to a composition which is an external preparation for skin.

### 2. Description of the Related Art

The change accompanied by skin aging such as fine wrinkles and sagging of skin is known to be caused by various factors including aging, ultraviolet rays, and the like.

In order to prevent or improving the symptoms of skin, various active components have been searched for, and compounding thereof has been examined. For example, ascorbic acid and derivatives thereof are known to be effective for improving symptoms of skin such as fine wrinkles or sagging of skin (JP2010-155837A).

Incidentally, carotenoid is known to have various physiological activities including an anti-oxidant function and the like. For example, Mol. Cancer Ther., 2005, Vol. 4(1), pp 177-186 describes the relationship between a certain type of cancer and carotenoid.

Moreover, stilbene-based compounds are known to inhibit keratosis of epidermal cells and to maintain growth of the cells (JP2004-091397A). Among the stilbene-based compounds, pterostilbene is known to have an anti-oxidant function (J. Biol. Chem., 2001, Vol. 276 (25), pp 22586-22594).

However, though research on the physiological activity of carotenoid and pterostilbene has been conducted, overall properties thereof remain unknown.

NOEVIR: "BioEssentials Supplement", December 2011, describes a micronutrient comprising antioxidants such as lycopene, and pterostilbene.

DATABASE GNPD [Online] MINTEL, September 2012, Anonymus: "Essence Destiny" describes a skin car composition formulated with astaxanthin, tomato fruit-origin nano lycopene and highly penetrative resveratrol to support ageing skin and firm the skin on the surface level and below.

US 2011/0274680 defines a synergistic chemical composition of bioactive compounds in a dietary supplement inter alia comprising pterostilbene and lycopene.

Yamashita E.: Fragrance Journal, vol. 34, no. 3, March 2006, pages 21-27 discusses cosmeceutical benefits of astaxanthin having antioxidant properties and being useful for visual wrinkle reduction.

WO 2011/051483 defines pterostilbene for use in the prevention and/or treatment of skin diseases. Optionally it is used in combination with QUER (3,3',4,4',5,6-pentahidroxiflavona). This composition may be used in the prevention and/or treatment of skin diseases, damages or injuries or of skin cancer.

### SUMMARY OF THE INVENTION

As described above, up to now, a composition, which is excellently effective for preventing or improving symptoms of skin such as fine wrinkles or sagging of skin, and an external preparation for skin have not been provided.

The present invention has been made in consideration of the above circumstances, and an object thereof is to provide a composition, which is excellently effective for preventing or improving symptoms of skin such as fine wrinkles or sagging skin, and an external preparation for skin containing the composition.

Means for achieving the above object is as follows.
[1] A composition containing astaxanthin and pterostilbene, in which a mass ratio between the astaxanthin and the pterostilbene is within a range from 300:1 to 1:300.
[2] The composition according to [1], in which the mass ratio between the astaxanthin and the pterostilbene is within a range from 30:1 to 1:30.

### DESCRIPTION OF THE PREFERRED EMBODIMENTS

Hereinafter, the present invention will be described in detail.

In the present specification, a range of numerical values that is described using "to" means a range that has numerical values listed before and after "to" as a lower limit and an upper limit respectively.

In the present specification, "aqueous phase" is used as a term contrast to "oil phase" regardless of the type of solvent.

In the present specification, a term "step" includes not only an independent step, but also a step which is not clearly distinguished from other steps as long as the step can achieve an intended object thereof.

In the present invention, when a composition contains plural substances corresponding to each component, unless otherwise specified, the amount of each component in the composition means a total amount of the plural substances contained in the composition.

### [Composition]

The composition of the present invention contains astaxanthin and pterostilbene. The astaxanthin and pterostilbene contained in the composition of the present invention are components that can function as active components having an effect of preventing or improving symptoms of skin such as fine wrinkles or sagging of skin. As a result, the composition of the present invention is effective for preventing or improving symptoms of skin such as fine wrinkles or sagging of skin.

Hereinafter, each of the components contained in the composition of the present invention will be described in detail.

### (Astaxanthin)

In the present invention, the astaxanthin includes astaxanthin and derivatives such as esters of astaxanthin. In the present invention, unless otherwise specified, these are collectively called "astaxanthin".

As the astaxanthin used in the present invention, any of the astaxathin as natural substances such as plants, algae, crustacean, bacteria and the astaxanthin obtained by common methods can be used.

The astaxanthin as a natural substance is obtained from, for example, red yeast phaffia, a green alga haematococcus, marine bacteria, or krill. Moreover, it can be obtained in the form of extract from cultures of these.

The astaxanthin may be contained in the composition of the present invention, as astaxanthin-containing oil obtained by being isolated from natural substances. Furthermore, the astaxanthin may be either a product obtained by appropriately purifying the astaxanthin-containing oil obtained by being isolated from natural substances if necessary or a synthetic product.

As the astaxanthin, in terms of quality or productivity, the astaxanthin extracted from an alga haematococcus (also referred to as "haematococcus alga extract") and a krill-derived dye are particularly preferable.

Specific examples of the haematococcus alga extract include extracts from Haematococcus pluvialis, Haematococcus lacustris, Haematococcus capensis, Haematococcus droebakensis, Haematococcus zimbabwiensis, and the like, but the present invention is not limited to these.

The haematococcus alga extract may be obtained by the method described in, for example, JP1993-68585A (JP-H05-68585A).

Moreover, a commercially available haematococcus alga extract can be used, and examples thereof include ASTOTS-S, ASTOTS-2,5 O, ASTOTS-5 O, ASTOTS-10 O, and the like manufactured by Takeda Shiki co., Ltd., Astereal Oil 50F, Astereal Oil 5F, and the like manufactured by Fuji Chemical Industry Co., Ltd., and BioAstin SCE7 and the like manufactured by Toyo Koso Kagaku Co., Ltd.

A content of a pure astaxanthin dye component in the haematococcus alga extract is preferably 0.001% by mass to 50% by mass, and more preferably 0.01% by mass to 25% by mass, from the viewpoint of handleability at the time of producing the composition.

The haematococcus alga extract usable in the present invention may contain astaxanthin as a pure dye component equivalent to a dye described in JP1990-49091A (JP-H02-49091A) or esters thereof In this case, it is possible to preferably use the haematococcus alga extract containing the esters generally in an amount of equal to or greater than 50 mol%, preferably in an amoun of equal to or greater than 75 mol%, and more preferably in an amount of equal to or greater than 90 mol%.

A content of the astaxanthin is preferably 0.00001% by mass to 0.2% by mass, more preferably 0.00005% by mass to 0.1% by mass, and even more preferably 0.0001% by mass to 0.05% by mass, with respect to a total mass of the composition.

### <<Pterostilbene>>

Pterostilbene (trans-1-(3,5-Diemthoxyphenyl)-2-(4-hydroxyphenyl)ethylene) is a compound represented by a chemical formula C₁₆H₁₆O₃.

The pterostilbene may be contained in the composition of the present invention, as pterostilbene-containing oil or pterostilbene-containing paste that is obtained by being isolated from natural substances.

In nature, the pterostilbene is contained in plants such as rabbiteye blueberry and the like. The pterostilbene-containing oil may be an extract that is obtained by being isolated from such natural substances. From the viewpoint of quality or productivity, it is particularly preferable for the pterostilbene to be extracted from an extract of bark of pterocarpus marsupium.

Moreover, the pterostilbene may be an extract, a product obtained by appropriately purifying an extract if necessary, or a synthetic product. The pterostilbene can be synthesized based on the method described in paragraph [0032] of JP2010-538077A and the like.

Furthermore, commercially available products can also be used as the pterostilbene. Examples of the products include Pterowhite from Sabinsa Japan Corporation, Pterostilbene from ChromaDex, Inc., and the like.

A content of the pterostilbene is preferably 0.00001% by mass to 0.2% by mass, more preferably 0.00005% by mass to 0.1% by mass, and even more preferably 0.0001% by mass to 0.05% by mass with respect to the total mass of the composition.

From the viewpoint of preventing or improving symptoms of skin such as fine wrinkles or sagging of skin, a mass ratio between the astaxanthin and the pterostilbene (astaxanthin:pterostilbene) is preferably 300:1 to 1:300, more preferably 30:1 to 1:30, and even more preferably 3:1 to 1:3.

### <Other Components>

The composition of the present invention can contain other components, according to the form of the composition, purpose, and the like. Examples of preferable other components include a functional oleaginous component, an emulsifier, other additive components, and the like.

### (Functional Oleaginous Component)

The functional oleaginous component is not particularly limited, as long as it is an oleaginous component which does not dissolve in an aqueous medium but dissolves in an oleaginous medium. The functional oleaginous components can be used by being appropriately selected from the components having physical properties suited for the purpose or the components having functionality. Examples of the functional oleaginous component include those used as an UV absorber, an anti-inflammatory, a moisturizer, a hair care product, a whitening agent, an anti-blemish agent, a cell activator, an emollient agent, a keratolytic agent, an anti-static agent, fat-soluble vitamins, a metabolic syndrome-improving agent, an antihypertensive, a sedative, and the like.

Examples of the functional oleaginous component include oils and fats such as ceramides including natural ceramides, sugar-modified ceramides like glycosphingolipid, and ceramide analogs, olive oil, camellia oil, macadamia nuts oil, castor oil, and coconut oil; hydrocarbon such as liquid paraffin, paraffin, Vaseline, ceresine, microcrystalline wax, and squalene; waxes such as carnauba wax, candelilla wax, jojoba oil, beeswax, and lanolin; esters such as isopropyl myristate, 2-octyldodecyl myristate, cetyl 2-ethylhexanoate, and diisostearyl malate; fatty acids such as pamitic acid, stearic acid, and isostearic acid; higher alcohols such as cetyl alcohol, stearyl alcohol, isostearyl alcohol, and 2-octyldodecanol; silicone oil such as methyl polysiloxane and methyl phenyl polysiloxane; fatty acid esters such as glycerin; fat-soluble vitamins such as vitamin E (tocopherol), vitamin A, and vitamin D; polymers; oil-soluble dyes such as carotenoids other than lycopene and xanthophyll; oil-soluble proteins; and the like. The examples also include various plant oils and animal oils as a mixture of these.

### (Surfactant)

The composition of the present invention may contain one, two, or more kinds of surfactants. The surfactant is a component which functions as an emulsifier when the composition of the present invention has a constitution of an emulsified composition or the like.

Examples of the surfactant include an anionic surfactant, a cationic surfactant, an amphoteric surfactant, and a nonionic surfactant.

From the viewpoint of emulsifying capacity, HLB of the surfactant is preferably equal to or higher than 10 and more preferably equal to or higher than 12. If HLB is too low, the emulsifying capacity becomes insufficient in some cases. Moreover, from the viewpoint of foam-suppressing effect, a surfactant with HLB equal to or higher than 5 but less than 10 may be concurrently used.

Herein, HLB refers to the balance between hydrophilicity and hydrophobicity that is generally used in the field of surfactant, and can be calculated using, for example, Kawakami's formula.

Among the surfactants, a nonionic surfactant is preferable since this is hypoallergenic and has a small effect on the environment. Examples of the nonionic surfactant include fatty acid esters of monosaccharide or polysaccharide, polyglycerin fatty acid esters, organic acid monoglyceride, propylene glycol fatty acid esters, polyglycerin condensed ricinoleic acid esters, sorbitan fatty acid esters, polyoxyethylene sorbitan fatty acid esters, and the like. Among these, it is possible to preferably use at least one kind selected from the fatty acid esters of monosaccharide or polysaccharide and the polygylcerin fatty acid esters.

Examples of the monosaccharide or polysaccharide constituting the fatty acid esters of monosaccharide or polysaccharide include glucose, sucrose, and the like.

As the fatty acid esters of monosaccharide or polysaccharide, esters consisting of monosaccharide or polysaccharide and a linear or brahced fatty acid having 6 to 22 carbon atoms are more preferable. Form the viewpoint of preparing a composition having high stability, sucrose myristic acid ester, sucrose palmitic acid ester, sucrose stearic acid ester, and sucrose oleic acid ester are even more preferable, and sucrose stearic acid ester is most preferable.

As the polyglycerin fatty acid esters, esters consisting of polyglycerin having a degree of polymerization of equal to or higher than 2 and a linear or branched fatty acid having 6 to 22 carbon atoms are more preferable.

From the viewpoint of stability, as the polyglycerin fatty acid esters, polyglyceryl-6 laurate, polyglyceryl-6 myristate, polyglyceryl-6 stearate, polyglyceryl-6 oleate, polyglyceryl-10 laurate, polyglyceryl-10 myristate, polyglyceryl-10 stearate, decaglyceryl monooleate, and polyglyceryl-10 oleate are more preferable, and decaglyceryl monooleate and decaglyceryl-10 oleate are most preferable.

### (Phospholipid)

The composition of the present invention may contain phospholipid. The phospholipid is a component which can function as an emulsifier when the composition of the present invention has a constitution of an emulsified composition.

Examples of the phospholipid include those extracted and isolated from biological bodies such as plants, animals, and microorganisms.

Specific examples of the phospholipid include various types of lecithin derived from plants such as soybean, corn, peanut, rape seeds, and barley, egg yolk, animals such as cow, microorganisms such as E. coli, and the like. Among these, egg yolk-derived lecithin or soybean-derived lecithin is preferable, and soybean-derived lecithin is more preferable.

Examples of compound name of the lecithin include glycerolecithin such as phosphatidic acid, phosphatidylglycerin, phosphatidylinositol, phosphatidylethanolamine, phosphatidyl methylethanolamine, phosphatidylcholine, phosphatidylserine, bisphosphaditic acid, diphosphatidylglycerin(cardiolipin), phosphatidylethanolamine, phosphatidylinositol, and lysophosphatidylcholine; sphingolecithin such as sphingomyelin; and the like.

Moreover, in the present invention, in addition to the above high-purity lecithin, hydrogenated lecithin, enzymatically decomposed lecithin, enzymatically decomposed hydrogenated lecithin, hydroxylecithin, and the like can be used. These lecithins usable in the present invention can be used singly, or can be used in the form of mixture consisting of plural kinds of the lecithins.

### (Other Additive Components)

The composition of the present invention may appropriately contain additive components, which are generally used in the field of food, cosmetics, and the like, according to the form of the composition.

Examples of the additive components include polyols such as glycerin and 1,3-butylene glycol; monosaccharides or polysaccharides such as kappa carrageenan, locust bean gum, guar gum, hydroxypropyl guar gum, xanthan gum, karaya gum, tamarind seed polysaccharide, gum Arabic, gum tragacanth, hyaluronic acid, sodium hyaluronate, sodium chondroitin sulfate, and dextrin; sugar alcohols such as sorbitol, mannitol, maltitol, lactose, maltotriitol, and xylitol; inorganic salts such as sodium chloride and sodium sulfate; proteins having a molecular weight of greater than 5,000, such as casein, albumin, methylated collagen, hydrolyzed collagen, water-soluble collagen, and gelatin; amino acids and derivatives thereof, such as glycin, alanine, valine, leucine, isoleucine, serine, threonine, aspartic acid, glutamic acid, cystine, methionine, lysine, hydroxylysine, arginine, histidine, phenylalanine, tyrosine, tryptophan, proline, hydroxyproline, and acetyl hydroxyproline; synthetic polymers such as a carboxyvinyl polymer, sodium polyacrylate, polyvinyl alcohol, polyethylene glycol, and an ethylene oxide-propylene oxide block copolymer; water-soluble cellulose derivatives such as hydroxyethyl cellulose-methyl cellulose; flavonoids (catechin, anthocyanin, flavone, isoflavon, flavane, flavanone, and rutin); ascorbic acid or derivatives thereof (ascorbic acid, sodium ascorbate, potassium ascorbate, calcium ascorbate, L-ascorbic acid phosphoric acid ester, ascorbyl magnesium phosphate, sodium ascorbyl phosphate, ascorbyl surfate, disodium ascorbyl sulfate salt, ascorbyl-2-glucoside, and the like); tocotrienol and derivatives thereof; phenolic acids (chlorogenic acid, ellagic acid, gallic acid, and propyl gallate); lignans; curcumins; coumarins; hydroxystilbene including pterostilbene; and the like. These may be contained in the composition, as a functional component, an excipient, a viscosity regulator, or a radical scanvenger for example, based on the function thereof.

In the present invention, for example, other additives such as various medicinal components, a pH regulator, a Ph buffer, a UV absorber, a preservative, a flavoring, and a colorant that are generally used for their own purpose may be concurrently used.

The form of the composition of the present invention is not particularly limited. The composition may be in the form of a composition containing a pharmaceutically acceptable carrier and other optional components which are used if necessary in addition to active components. Examples of the form of the composition include an oil composition, an emulsified composition, and the like. The emulsified composition is preferably an oil-in-water type emulsified composition.

### -Method for Producing Composition-

The method for producing the composition of the present invention is not particularly limited, and can be appropriately selected according to the intended formulation.

For example, when the composition of the present invention is prepared as an emulsified composition, examples of the method for producing the emulsified composition include emulsification in water, emulsification in oil, alternating addition, and the like that are generally used. Moreover, in the case of the oil-in-water type emulsified composition, either an emulsification method using a general emulsification device (for example, a stirrer, an impeller stirrer, a homomixer, or a continuous flow type shearing device) which utilizes shearing action or a two-stage emulsification method in which two or more kinds of emulsification devices are concurrently used in a method of emulsifying the composition through a high-pressure homogenizer, an ultrasonic disperser, or the like in addition to the one-stage emulsification operation in which the composition is emulsified by the aforementioned emulsification devices may be used.

Furthermore, as a widely used emulsification method, a method of using mechanical force, that is, a method of dispersing oil droplets by means of applying strong shearing force from the outside is used. In order to obtain the mechanical force, a high-speed and high-shear stirrer is most generally used. As such a stirrer, stirrers called a homomixer, a disper mixer, and an ultramixer are commercially available.

As another mechanical emulsification device that is useful for atomization, a high-pressure homogenizer may be used. The high-pressure homogenizer can apply stronger shearing force compared to the stirring method, and accordingly, the composition can be atomized even if the amount of emulsifier is relatively small.

As an emulsification device which is a disperser having relatively excellent energy efficiency and has a simple structure, there is an ultrasonic homogenizer.

Moreover, as another known emulsifying means, it is also effective to use a method of using a static mixer, a microchannel, a micromixer, a membrane emulsification device, or the like which does not include an external stirring portion and requires low energy.

In the present invention, the temperature condition at the time of emulsification and dispersion is not particularly limited. However, from the viewpoint of stability of the functional oleaginous component, the temperature is preferably 10°C to 100°C. A preferable range of the temperature can be appropriately selected according to a melting point of the functional oleaginous component to be handled.

Moreover, when a high-pressure homogenizer is used in the present invention, the pressure is preferably equal to or higher than 50 MPa, more preferably 50 MPa to 280 MPa, and even more preferably 100 MPa to 280 MPa.

Furthermore, from the viewpoint of maintaining a particle size of dispersed particles, it is preferable for the emulsion as the composition having undergone emulsification and dispersion to be cooled by passing through any type of cooler within 30 seconds and preferably within 3 seconds immediately after passing through a chamber.

The composition of the present invention having an action of improving symptoms of skin such as fine wrinkles or sagging of skin. Specifically, the composition is expected to exert a local effect by being locally administered to skin as an external preparation for skin. The composition is also expected to exert an effect in a wide range by being orally administered and absorbed into the body as a functional food.

### [External Preparation for Skin]

The external preparation for skin may contain the composition containing the astaxanthin or the pterostilbene. If necessary, the external preparation for skin can further contain other components such as a cell activator, an anti-inflammatory, and other additives.

The external preparation for skin can be applied to cosmetics, percutaneous pharmaceutical products, and the like.

Examples of the cosmetics to which the external preparation for skin is applied include cosmetics for skin care (a toner, a serum, an emulsion, a cream, and the like), lipstick, cosmetics as a sunscreen, cosmetics for makeup, and the like, but the examples are not limited to these.

The external preparation for skin may be produced based on the aforementioned method for preparing the composition.

When the external preparation for skin is used particularly for aqueous products such as a toner, a serum, an emulsion, a cream, a pack, a mask pack, cosmetics for washing hair, cosmetics for flavoring, a liquid body washer, cosmetics for UV care, cosmetics as a deodorant, and cosmetics for oral care (in the field of cosmetics), a product having transparency is obtained. Moreover, it is possible to inhibit occurrence of problems such as precipitation, sedimentation, and neck ring that are caused under harsh conditions such as long-term storage or a sterilization process.

The amount of the composition added to the external preparation for skin cannot be uniformly specified and varies with the type of the form of preparation, purpose, and the like. However, the amount is 0.01% by mass to 10% by mass and preferably 0.05% by mass to 5% by mass, with respect to the amount of product. If the amount of the composition added is equal to or greater than 0.01% by mass, the product is expected to be able to exert the intended effect, and if it is equal to or smaller than 10% by mass, an appropriate effect can be efficiently exerted.

### Examples

Hereinafter, examples of the present invention will be described in detail, but the present invention is not limited thereto. Moreover, unless otherwise specified, "part(s)" and "%" are based on mass.

### [Test Example 1]

Various hydroxystilbenes shown in the following Table 1 were dissolved in ethanol, thereby preparing a 1% solution. The solution was stirred together with PEG-60 hydrogenated castor oil which was in an amount 10 times the amount of the solution with respect to 1 part of the ethanol solution. Thereafter, the resultant was diluted by 100-fold with a citric acid buffer solution, thereby preparing a sample.

pH of the sample prepared as above was regulated to be 8, and the sample was left as is for a month and a half (43 days) at room temperature. Thereafter, by means of HPLC, a residual amount of various hydroxystilbenes in each sample was determined. The results are shown in Table 1. Analysis conditions for HPLC are as follows.

### (Analysis conditions for HPLC)

Column: C18 5 µm, 4.6 mm × 250 mm
Flow rate: 1 ml/min
Column temperature: 40°C
Detection wavelength: 310 nm

Developing solution: acetonitrile/water = 20/80 (volume ratio, containing 0.1% by volume of acetic acid and 0.1% by volume of triethanolamine)

**[Table 1]**

| Hydroxystilbene | CAS No. | Residual amount (%) |
|---|---|---|
| Resveratrol | 501-36-0 | 54 |
| Trans-resveratol (trans-resveratol 4'-O-beta-D-glucuronide) | 38963-95-0 | 73 |
| Pterostilbene | 537-42-8 | 99 |
| Rhapontigenin | 500-65-2 | 50 |
| (E)-3,4',5-trihydroxy-3'-methoxystilbene | 32507-66-7 | 46 |
| Piceatannol | 10083-24-6 | 26 |

Among the stilbenes, the hydroxystilbenes having a hydroxyl group is particularly excellent in antioxidant properties. On the contrary, it is considered that due to the antioxidant properties, the compound itself becomes unstable.

However, the above results clearly showed that among the hydroxystilbenes, pterostilbene is practically not decomposed over time.

### [Example 1, Reference Examples 2, 3 and Comparative Examples 1 to 9]

The components shown in Table 2 were mixed and stirred together by using a magnetic stirrer (manufactured by AS ONE Corporation) at a compositional ratio shown in the table, thereby preparing respective compositions.

Each of the composition was evaluated in terms of fine wrinkle-improving effect.

A test was performed on a group consisting of ten males. The males were asked to apply the composition of comparative example to the corner of their left eye and to apply the composition of example to the corner of their right eye, twice a day in the morning and evening.

After continuous application for 1 month, images taken before the test were compared to images taken after the test so as to evaluate the fine wrinkle-improving effect according to improvement score shown in Table 3. In addition, a survey was taken regarding the symptom of pigmentation as shown in Table 4, and evaluation was conducted. The results are shown in Table 5.

**[Table 2] (Total amount: 100% by mass)**

| | | Example 1 | Example 2 * | Example 3 * | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 |
|---|---|---|---|---|---|---|---|---|---|---|---|---|---|
| | astaxanthin | 0.05 | - | - | - | - | 0.05 | - | - | - | 0.5 | - | - |
| | Lycopene | - | 0.05 | - | - | - | - | 0.05 | - | - | - | 0.5 | - |
| | Lutein | - | - | 0.05 | - | - | - | - | 0.05 | - | - | - | 0.5 |
| | Pterostilbene | 0.05 | 0.05 | 0.05 | - | 0.05 | - | - | - | 0.5 | - | - | - |
| | Glycerin | 5 | 5 | 5 | 5 | 5 | 5 | 5 | 5 | 5 | 5 | 5 | 5 |
| | 1,3-butyleneglycol | 6.5 | 6.5 | 6.5 | 6.5 | 6.5 | 6.5 | 6.5 | 6.5 | 6.5 | 6.5 | 6.5 | 6.5 |
| | Polyoxyethylene sorbitan monolauric acid ester | 1.2 | 1.2 | 1.2 | 1.2 | 1.2 | 1.2 | 1.2 | 1.2 | 1.2 | 1.2 | 1.2 | 1.2 |
| | Ethanol | 6 | 6 | 6 | 6 | 6 | 6 | 6 | 6 | 6 | 6 | 6 | 6 |
| | Tocopherol acetate | 0.001 | 0.001 | 0.001 | 0.001 | 0.001 | 0.001 | 0.001 | 0.001 | 0.001 | 0.001 | 0.001 | 0.001 |
| | Magnesium ascorbyl phosphate | 0.2 | 0.2 | 0.2 | 0.2 | 0.2 | 0.2 | 0.2 | 0.2 | 0.2 | 0.2 | 0.2 | 0.2 |
| | Xanthan gum | 0.1 | 0.1 | 0.1 | 0.1 | 0.1 | 0.1 | 0.1 | 0.1 | 0.1 | 0.1 | 0.1 | 0.1 |
| | Carboxyvinyl polymer | 0.1 | 0.1 | 0.1 | 0.1 | 0.1 | 0.1 | 0.1 | 0.1 | 0.1 | 0.1 | 0.1 | 0.1 |
| | Decaglyceryl monooleate | 0.2 | 0.2 | 0.2 | 0.2 | 0.2 | 0.2 | 0.2 | 0.2 | 0.2 | 0.2 | 0.2 | 0.2 |
| | Sucrose stearic acid ester | 0.1 | 0.1 | 0.1 | 0.1 | 0.1 | 0.1 | 0.1 | 0.1 | 0.1 | 0.1 | 0.1 | 0.1 |
| | Citric acid | 0.05 | 0.05 | 0.05 | 0.05 | 0.05 | 0.05 | 0.05 | 0.05 | 0.05 | 0.05 | 0.05 | 0.05 |
| | Sodium citrate | 0.1 | 0.1 | 0.1 | 0.1 | 0.1 | 0.1 | 0.1 | 0.1 | 0.1 | 0.1 | 0.1 | 0.1 |
| | Collagen | 1 | 1 | 1 | 1 | 1 | 1 | 1 | 1 | 1 | 1 | 1 | 1 |
| | N-acetylhydroxypro line | 1 | 1 | 1 | 1 | 1 | 1 | 1 | 1 | 1 | 1 | 1 | 1 |
| | Methyl paraoxybenzoate | 0.02 | 0.02 | 0.02 | 0.02 | 0.02 | 0.02 | 0.02 | 0.02 | 0.02 | 0.02 | 0.02 | 0.02 |
| | Rose extract oil | - | - | - | - | - | - | - | - | - | - | - | - |
| | Rose extract water | 0.001 | 0.001 | 0.001 | 0.001 | 0.001 | 0.001 | 0.001 | 0.001 | 0.001 | 0.001 | 0.001 | 0.001 |
| | Flavoring | Appropriate amount | Appropriate amount | Appropriate amount | Appropriate amount | Appropriate amount | Appropriate amount | Appropriate amount | Appropriate amount | Appropriate amount | Appropriate amount | Appropriate amount | Appropriate amount |
| | Polyoxyethylene hydrogenated castor oil | 0.1 | 0.1 | 0.1 | 0.1 | 0.1 | 0.1 | 0.1 | 0.1 | 0.1 | 0.1 | 0.1 | 0.1 |
| | Purified water | Balance | Balance | Balance | Balance | Balance | Balance | Balance | Balance | Balance | Balance | Balance | Balance |

| | | | | | | | | | | | | | |
|---|---|---|---|---|---|---|---|---|---|---|---|---|---|
| * Reference | | | | | | | | | | | | | |

**[Table 3]**

| Score | Condition of fine wrinkles |
|---|---|
| 4 | Markedly improved |
| 3 | Moderately improved |
| 2 | Mildly improved |
| 1 | Slightly improved |
| 0 | No change |
| -1 | Slightly worsened |

**[Table 4]**

| Score | Symptom of skin pigmentation |
|---|---|
| 4 | Markedly improved |
| 3 | Moderately improved |
| 2 | Mildly improved |
| 1 | Slightly improved |
| 0 | No change |
| -1 | Slightly worsened |

| | |
|---|---|
| * Reference | |

**[Table 5]**

| | Fine wrinkle-improving effect | | pigmentation | |
|---|---|---|---|---|
| | Mean | SD | Mean | SD |
| Example 1 | 3.4 | 0.8 | 3.2 | 1.0 |
| Example 2* | 3.1 | 1.1 | 3.0 | 1.2 |
| Example 3* | 3.2 | 0.8 | 3.3 | 0.8 |
| Comparative Example 1 | 1.0 | 0.9 | 0.8 | 0.8 |
| Comparative Example 2 | 0.8 | 0.9 | 1.4 | 0.7 |
| Comparative Example 3 | 1.5 | 0.7 | 1.3 | 0.5 |
| Comparative Example 4 | 1.1 | 0.7 | 1.1 | 0.7 |
| Comparative Example 5 | 1.2 | 0.6 | 1.3 | 0.5 |
| Comparative Example 6 | 2.0 | 1.1 | 1.9 | 1.1 |
| Comparative Example 7 | 1.7 | 1.4 | 2.1 | 1.0 |
| Comparative Example 8 | 0.8 | 0.9 | 1.9 | 0.9 |
| Comparative Example 9 | 1.3 | 0.9 | 1.8 | 0.9 |

| | | | | |
|---|---|---|---|---|
| * Reference * SD: Standard Deviation | | | | |

The composition of the present invention exerted a fine wrinkle-improving effect.

### [Example 4]

NPκB is considered to be involved in skin aging at an early stage. Therefore, an NFκB intranuclear transition inhibition rate of carotenoid and pterostilbene was examined.

Normal human dermal fibroblasts (HDF cells, KURABO INDUSTRIES LTD.) were subcultured three times according to a common method, in a GlutaMax medium (D-MEM, Gibco) supplemented with 10% fetal bovine serum (FBS, Gibco). The subcultured HDF cells were caused to adhere to a container at 1,000,000 cells/25 cm² and put in a serum starvation state for a day. Thereafter, the HDF cells were washed with phosphate buffered saline (PBS).

The cells were irradiated with UVB (20 mJ) emitted from right above the cells, and then the medium was replaced with a medium obtained by adding 0.1% FBS to D-MEM containing the carotenoid and the pterostilbene shown in Tables 6 and 7.

6 hours after the replacement of the medium, nuclei were extracted using NucBuster (Novagen), and the amount of NF-κB moved into the nuclei was determined by Western blotting (WB) (primary antibody: NF-κB p65 Rabbit Monoclonal Antibody (Cat. #1546-1), EPITOMICS).

The results are shown in Tables 6 and 7. Moreover, in Experiment 1 and Experiment 7, for comparison, the cells were not irradiated with UVB.

**[Table 6]**

| | UV | Pterostilbene | Concentration (ppm) | Carotenoid | Concentration (ppm) | Ratio of amount of NF-κB moving into nucleus |
|---|---|---|---|---|---|---|
| Experiment 1 | Not irradiated | None | - | None | - | 100 % |
| Experiment 2 | Irradiated | None | - | None | - | 684% |
| Experiment 3 | Irradiated | Pterostilbene | 5 | None | - | 420% |
| Experiment 4 | Irradiated | Pterostilbene | 5 | Astaxanthin | 15 | 120% |
| Experiment 5 * | Irradiated | Pterostilbene | 5 | lycopene | 15 | 110 % |
| Experiment 6* | Irradiated | Pterostilbene | 5 | Lutein | 15 | 130% |

| | | | | | | |
|---|---|---|---|---|---|---|
| * Reference | | | | | | |

**[Table 7]**

| | UV | Pterostilbene | Concentration (ppm) | Carotenoid | Concentration (ppm) | Ratio of amount of NF-κB moving into nucleus |
|---|---|---|---|---|---|---|
| Experiment 7 | Not irradiated | None | - | None | - | 100% |
| Experiment 8 | Irradiated | None | - | None | - | 684% |
| Experiment 9 | Irradiated | Pterostilbene | 5 | astaxanthin | 1500 | 97% |
| Experiment 10 | Irradiated | Pterostilbene | 5 | astaxanthin | 150 | 102% |
| Experiment 11 | Irradiated | Pterostilbene | 5 | astaxanthin | 15 | 120% |
| Experiment 12 | Irradiated | Pterostilbene | 5 | astaxanthin | 1.5 | 145% |
| Experiment 13 | Irradiated | Pterostilbene | 5 | astaxanthin | 0.15 | 190% |
| Experiment 14 | Irradiated | Pterostilbene | 5 | astaxanthin | 0.015 | 248% |

By combining the carotenoid with the pterostilbene at various quantitative ratios, the amount of NF-κB that moved into a nucleus due to UV irradiation was reduced. The result clearly shows that the composition according to the present invention can prevent or improve skin aging at an early stage.

Examples 5 to 7 show examples of the formulation of the external preparation for skin that uses the composition of the present invention.

As the magnesium ascorbyl phosphate used in the formulatioin, C-Mate manufactured by BASF Japan Ltd., NIKKOL VC-PMG manufactured by Nikko Chemicals Co., Ltd., and the like can be used.

### [Example 5] Serum

A serum having the following composition was prepared by a common method (a total mass of 100% by mass).

### <Composition>

| (% by mass) | |
|---|---|
| Magnesium ascorbyl phosphate | 0.2 |
| Glycerin | 4.0 |
| 1,3-butyleneglycol | 8.0 |
| Betaine | 0.5 |
| Polyglyceryl-2 stearate | 0.5 |
| Xanthan gum | 0.2 |
| Hydrolyzed elastin | 0.1 |
| Royal jelly extract | 0.1 |
| Glucosyl rutin | 0.1 |
| Acetyl hydroxyproline | 1.0 |
| Water-soluble collagen | 1.0 |
| Hydrolyzed collagen | 1.0 |
| Haematococcus pluvialis oil (containing astaxanthin) | 0.05 |
| Tocopherol | 0.15 |
| Lecithin | 0.1 |
| Polyglyceryl-10 oleate | 0.2 |
| Sucrose stearate | 0.2 |
| Glycery tri(caprylate/caprate) | 0.2 |
| Tomato extract | 0.05 |
| Extract of bark of pterocarpus marsupium (containing pterostilbene) | 0.1 |
| Phenoxyethanol | 0.2 |
| Iodopropynyl butyl carbamate | 0.01 |
| Hydroxypropyl cyclodextrin | 0.03 |
| Pentylene glycol | 0.2 |
| Damascus rose oil | appropriate amount |
| Flavoring | appropriate amount |
| PEG-60 hydrogenated castor oil | 0.2 |
| Citric acid | 0.2 |
| Sodium hydroxide | appropriate amount |
| Purified water | balance |

### [Example 6] Whitening Serum

A whitening serum having the following composition was prepared by a common method (a total mass of 100% by mass).

### <Composition>

| (% by mass) | |
|---|---|
| Magnesium ascorbyl phosphate | 2.0 |
| Dipotassium glycyrrhizate | 0.2 |
| Glycerin | 4.0 |
| 1,3-butyleneglycol | 5.0 |
| Sodium pyrosulfite | 0.1 |
| Alcaligenes latus B-16 polymer | 0.05 |
| Xanthan gum | 0.1 |
| Hydrolyzed yeast extract solution | 0.1 |
| Yeast extract | 0.1 |
| Acetyl hydroxyproline | 1.0 |
| Water-soluble collagen | 1.0 |
| Hydrolyzed collagen | 1.0 |
| Krill extract (containing astaxanthin) | 0.05 |
| Natural vitamin E | 0.1 |
| Soybean phospholipid | 0.05 |
| Polyglyceryl monooleate | 0.1 |
| Sucrose fatty acid ester | 0.1 |
| Extract of bark of pterocarpus marsupium (containing pterostilbene) | 0.1 |
| Centella asiatica extract | 0.1 |
| Water-soluble Centella asiatica extract | 0.1 |
| Phenoxyethanol | 0.3 |
| 1,2-pentanediol | 0.2 |
| Methyl paraoxybenzoate | 0.05 |
| Flavoring | appropriate amount |
| Polyoxyethylene hydrogenated castor oil | 0.2 |
| Citric acid | 0.2 |
| Sodium hydroxide | appropriate amount |
| Purified water | balance |

### [Example 7] Whitening Toner

A whitening toner having the following composition was prepared by a common method (a total mass of 100% by mass).

### <Composition>

| (% by mass) | |
|---|---|
| Magnesium ascorbyl phosphate | 1.0 |
| Arbutin | 1.0 |
| Dipotassium glycyrrhizate | 0.2 |
| Glycerin | 3.0 |
| 1,3-butyleneglycol | 5.0 |
| Polyoxyethylene methyl glucoside | 1.0 |
| Polyethylene glycol 1540 | 1.0 |
| Sea algae extract | 0.1 |
| Sodium hyaluronate | 0.1 |
| Acetyl hydroxyproline | 1.0 |
| Water-soluble collagen | 1.0 |
| Hydrolyzed collagen | 1.0 |
| Krill extract (containing astaxanthin) | 0.05 |
| Natural vitamin E | 0.1 |
| Soybean phospholipid | 0.05 |
| Polyglyceryl monooleate | 0.1 |
| Sucrose fatty acid ester | 0.1 |
| Extract of bark of pterocarpus marsupium (containing pterostilbene) | 0.01 |
| Centella asiatica extract | 0.1 |
| Phenoxyethanol | 0.3 |
| Ethylhexyl glycerin | 0.5 |
| Flavoring | appropriate amount |
| Polyoxyethylene hydrogenated castor oil | 0.2 |
| Citric acid | 0.2 |
| Sodium hydroxide | appropriate amount |
| Purified water | balance |

## Claims

1. A composition which is an external preparation for skin, comprising:
astaxanthin; and
pterostilbene,
wherein the mass ratio between the astaxanthin and the pterostilbene is within a range from 300:1 to 1:300.

2. The composition according to claim 1,
wherein the mass ratio between the astaxanthin and the pterostilbene is within a range of 30:1 to 1:30.

## Patentansprüche

1. Zusammensetzung, die ein externes Präparat für die Haut ist, enthaltend:
Astaxanthin und
Pterostilben,
worin das Massenverhältnis zwischen dem Astaxanthin und dem Pterostilben innerhalb eines Bereiches von 300:1 bis 1:300 ist.

2. Zusammensetzung nach Anspruch 1, worin das Massenverhältnis zwischen dem Astaxanthin und dem Pterostilben innerhalb eines Bereiches von 30:1 bis 1:30 ist.

## Revendications

1. Composition qui est une préparation externe pour la peau, comprenant :
de l'astaxanthine ; et
du ptérostilbène,
dans laquelle le rapport de masse entre l'astaxanthine et le ptérostilbène est dans une plage de 300:1 à 1:300.

2. Composition selon la revendication 1,
dans laquelle le rapport de masse entre l'astaxanthine et le ptérostilbène est dans une plage de 30:1 à 1:30.
